# EUROPEAN PATENT APPLICATION

(11) **EP 2 666 440 A2**
(43) Date of publication of application: **27.11.2013**
(21) Application number: 12197590.8
(22) Date of filing: 17.12.2012
(51) Int. Cl.: A61F 2/00

(54) **System and method for repairing muscle defect**

(30) Priority: 15.12.2011 US 201113326696; 19.04.2012 US 201213450676
(71) Applicant: Insightra Medical, Inc., Irvine, California 92618 (US)
(72) Inventor: Noda, Wayne A., California, CA California 92692 (US); Adzich, Vaso, California, CA California 92705 (US); Bell, Stephen Graham, 00142 Roma (IT); Amato, Giuseppe, 90144 Roma (IT)
(74) Representative: Schütte, Gearoid

(57) **Abstract**

A space in a muscle wall is dilated by a plunger-based mechanism to break up fibrotic bands by divulsion. While the space is dilated a dynamic plug is advanced into it, with the plug expanding and contracting with the space.

## Description

### Field of the Invention

The present invention relates generally to the repair of defects in muscular structures.

### Background of the Invention

A hernia is one example of a defect in a muscle wall that can be established by a hole or opening in the wall. The above-referenced patent applications provide devices and methods for advancing a prosthetic implant into a hernial opening to repair the opening. As understood herein, the dynamic implant disclosed in one or more of the above-referenced applications has fulfilled a long-felt but unaddressed need in surgery, namely, by overcoming deficiencies in older static, passive prosthetics used for hernia repair that do not move in harmony with the dynamic elements of the groin. In contrast, the flexible implant structure creates a scaffold-like structure with a leaf spring-like force to foster tissue ingrowth, promoting tissue regeneration to form an integral barrier against protrusion. New vascular structures are formed within the mesh and/or fibers, a significant improvement over conventional non-biologic implants that only facilitate ingrowth of stiff, regressive, and dense fibrotic scar tissue that leads to chronic pain. As further recognized herein, such an advantageous implant may be used to repair other muscular defects such as holes or openings in muscle walls.

### Summary of the Invention

According to the invention, there is provided a method which includes providing a mesh body established by plural mesh strands, the mesh body being engaged with at least one strengthening member that is not a mesh strand, deforming the mesh body to a first configuration in which the mesh body can be advanced into a hole in a body tissue, advancing the mesh body into the hole, and allowing the mesh body to assume a second configuration at least partially under influence of the strengthening member in which the mesh body expands to substantially fill the hole, the mesh body creating a scaffold-like structure with a leaf spring-like force to foster tissue ingrowth and promote tissue regeneration such that new vascular structures can be formed within the mesh while minimizing ingrowth into the mesh body of fibrotic scar tissue. The hole need not be an inguinal canal hernia. Note that "hole" can mean loss/absence of tissue mass from disease, trauma, hereditary, necrosis from poison bites, etc.

In some implementations the hole is dilated prior to advancing the mesh body into the hole. The mesh body can be advanced into the hole using open surgery, or endoscopic surgery, or laparoscopic surgery, or natural orifice surgery, or some combination thereof.

In example embodiments the body tissue affected by the hole is defined at least in part by a patient's pelvis floor, or by a patient's long extremity muscle such as a calf or bicep, or by a patient's heart, a patient's heart septum, a patient's abdominal wall, or a patient's diaphragm a patient's femoral canal. Or, the body tissue affected by the hole is defined at least in part by a non-muscle organ such as the liver, kidney, lung, or pancreas.

In one embodiment, the mesh body is petal-shaped. In an example, the implant body includes plural petal-shaped lobes.

In another aspect, a method of the invention includes providing a mesh body established by plural mesh strands, deforming the mesh body to a first configuration in which the mesh body can be advanced into a hole or defect in a body tissue, advancing the mesh body into the hole, and allowing the mesh body to assume a second configuration in which the mesh body expands to substantially fill the hole, the mesh body creating a scaffold-like structure with a leaf spring-like force to foster tissue ingrowth and promote tissue regeneration such that new vascular structures can be formed within the mesh while minimizing ingrowth into the mesh body of fibrotic scar tissue, wherein the body tissue is defined at least in part by a patient's long extremity muscle, or by a patient's heart, or by a patient's abdominal wall, or by a patient's diaphragm, or by a patient's femoral canal, or by a patient's liver, or by a patient's kidney, or by a patient's lung, or by a patient's pancreas.

In a further aspect, the invention provides apparatus for carrying out the method as described herein.

### Brief Description of the Drawings

The details of the present invention, both as to its structure and operation, can best be understood in reference to the accompanying drawings, in which like reference numerals refer to like parts, and in which:
Figure 1 is a perspective view of an example mesh in the insertion configuration about to be advanced through a hole in a muscle wall, with the plug portion removed for clarity;
Figure 2 is an elevational view of the mesh in the implanted configuration positioned against the posterior surface of the wall blocking the hole, which is shown in phantom;
Figure 3 is a first perspective view of an example dilator, showing the plunger through the transparent barrel in the advanced position with an example implant schematically shown having been pushed out of the distal end;
Figure 4 is a second perspective view of the example dilator, showing the plunger in the retracted position;
Figure 5 is a plan view of the proximal end of the barrel;
Figure 6 is a first side view of the example dilator, showing the plunger in the advanced position;
Figure 7 is a second side view of the example dilator, showing the plunger in the retracted position;
Figure 8 is a first end view of the example dilator;
Figure 9 is a second end view of the example dilator;
Figure 10 is a perspective view of an example implant having a mesh with integral plug portion woven into the mesh, with both the mesh and plug including strengthening members, showing two enlarged views to illustrate the strengthening members;
Figure 11 is a side view of an alternate example dilator with a movable stop flange in the retracted position, showing some structure in phantom; and
Figure 12 is a side view of the alternate example dilator shown in Figure 11 in the advanced position.

### Detailed Description of the Preferred Embodiment

As set forth in greater detail below, methods and devices are disclosed to repair various muscle walls. A dilating instrument can be used to rupture fibrous bands within the muscle wall to allow a return to the natural physiological state of contraction/relaxation. The dilating instrument may be engaged with a preloaded implant and if desired a force gauge mechanism (e.g. manometer) to precisely control the dilation forces.

A dynamic implant can then be deployed in the muscle wall that contracts and relaxes with the muscle wall. In addition, in non-limiting embodiments adhesions may be resolved to further release the muscle wall prior to dilation by, e.g., scrubbing the adhesions with a pad. When the delivery device is removed the newly restored contractibility of the muscle usually causes the muscle to grasp and hold the implant.

In addition to physical dilation of the defect, the fibrous bands can also be disrupted using mechanical, electrical, thermal, magnetic, or chemical processes. For example, direct or indirect stimulation by electricity of the muscles can be used to create deform spasms which through non-physiologic contraction break the fibrosis. Cooling or heating may be used at temperatures established to break the stiff connective fibers only within the muscles, thus breaking the fibrosis. Magnetic objects such as small magnetic or ferromagnetic discs or even small particles can be disposed in the muscle through, e.g., a catheter and then a large magnet disposed external to the patient to attract the internal magnetic objects to stretch the muscles and cause divulsion. Enzymatic or chemical lysis of stiff connective fibers can be applied through, e.g., hyperdermic injection or perioperatively to break down only fibrotic tissue and not healthy tissue.

As described further below, the present implant preferably is a dynamic device that expands and contracts in a radial fashion in synchrony with the muscles of the wall to allow the contraction and relaxation in a normal physiological way. The implant is configured to exert a constant outward pressure on the surrounding tissue, and it may incorporate a unique lamellar structure at its core. The implant may be made of materials that have low tissue reactivity. A solid, liquid gel or gas or combination of any to create the implant and can be made from autologous tissues, xenotissues or any form of biological material. The implant can be permanent, temporary, absorbable, non-absorbable or a combination of all of these and can be detected radiologically or by using other imaging diagnostic procedures. The implant can be, e.g., tubular, round, conical, irregular, regular etc. The implant may be retained within the defect by friction, i.e., by radial force between the implant and muscle wall. Also, the implant may be sutured, clipped, or glued to the wall.

Application of the implant may use open surgery, laparoscopic surgery, endoscopic surgery, natural orifice surgery, or a combination thereof. The implant can be deployed forwards or backwards, and the applicator can be temporary, permanent, non-absorbable, or absorbable.

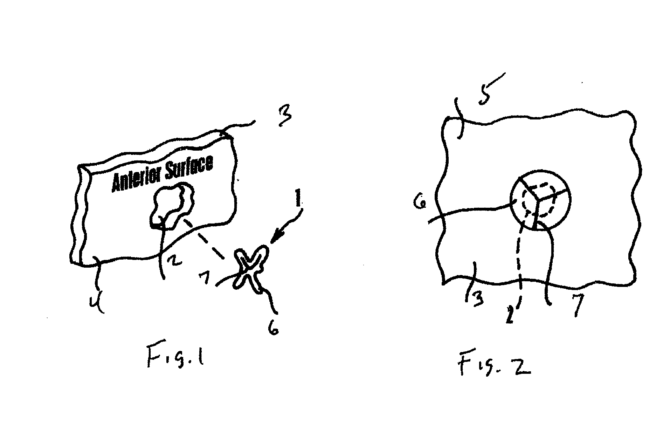

Referring now to Figures 1 and 2, a device is shown, generally designated 1, for blocking an opening 2 of a muscle wall 3 that may have an anterior surface 4 and a posterior surface 5. As shown, the device 1 includes a mesh body 6 and one or more stiffening members 7 for fortifying the body 6. In an example, the device 1 may be established by any of the specific embodiments described below. In other examples the device 1 may be established by any of the implants described in the above-referenced patent applications.

Without limitation the mesh can be made of polypropylene, expanded polytetrafluoroethylene (PTFE), polyester, biodegradable materials, the material marketed as "dualmesh", a trademark of W.L. Gore, or even metal such as stainless steel or nitinol, or some combination thereof.

The device 1 can be moved between an insertion configuration (Figure 1), in which the device 1 is smaller than the hole 2 to facilitate advancing the device 1 into the hole 2, and an implanted configuration (Figure 2), in which the device is implanted in the hole 2 and a portion of the device 1 is substantially flat and unwrinkled/unfolded and is larger than the hole 2 to block the hole 2. As will be clearer after disclosure below, the device 1 is biased to the implanted configuration at least by the strengthening member 7 and in some implementations by the mesh body 6 as well. Thus, the device 1 is resilient and is materially biased to the implanted configuration.

The wall 3 may be, as an example, a wall of an abdomen muscle in which the hole 2 has formed as a hernia. Typically, the device 1 may be deformed to the insertion configuration, advanced through the hole 2 from the anterior surface 4 until it clears the hole 2, and then permitted (as by releasing the device 1) to assume the implanted configuration in which a portion of the device 1 lies flat against the posterior surface 5 of the wall 3, blocking the hole 2. Defects in other muscle walls may be similarly resolved using the device 1. Other muscle wall defects such as pelvic floor prolapse may be similarly resolved.

With more particularity, the hole 2 or other defect may be in the pelvis floor or diaphragm, including the levitor ani and coccygeus. Or, the muscle with hole or defect 2 to be repaired may be a long extremity muscle such as biceps or a calf muscle, in which case the defect 2 may be a myotendineal lesion or rupture. Similar traumatic muscular ruptures around the body may be repaired. Note that the defect may be caused by tissue removal incident to removing a tumor or by tissue destroying infections. Yet again, the hole 2 may be a myocardial defect, either congenital, interatrial, or interventricular, in the heart. The defect may be in the heart as a result of necrosis or myocardial ischemia. Or, the defect 2 may be a trans-septal cardiovascular defect, in which case the muscle to be repaired is a septum of the heart. The defect may be in the anterior abdominal wall, or the diaphragm, or the femoral canal.

Indeed, the hole or defect 2 may be in a non-muscle organ such as the liver, kidney, lung, or pancreas in which the benefits noted above may provide regenerative results.

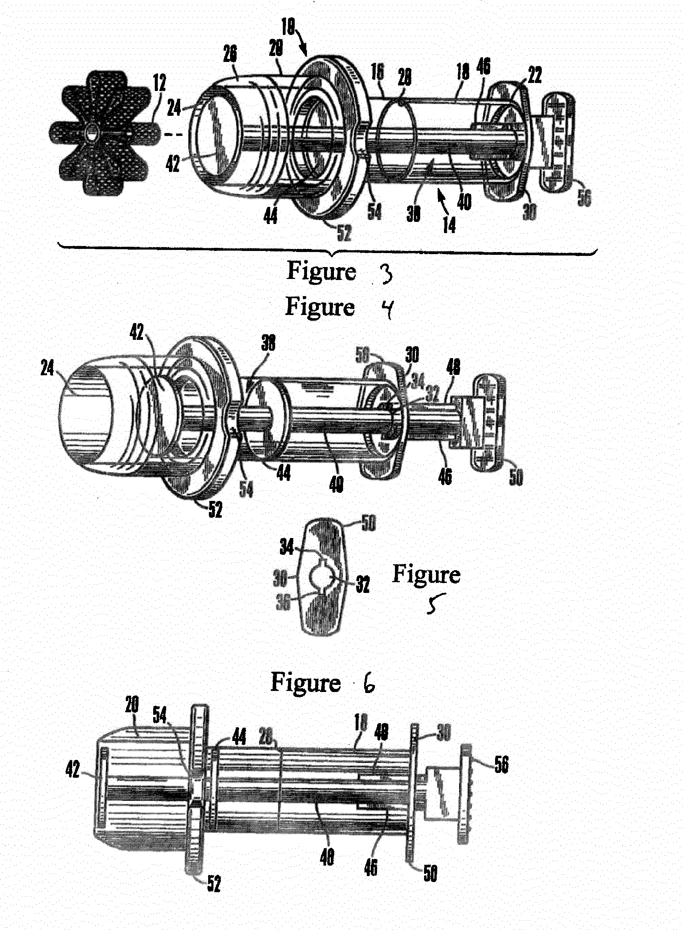

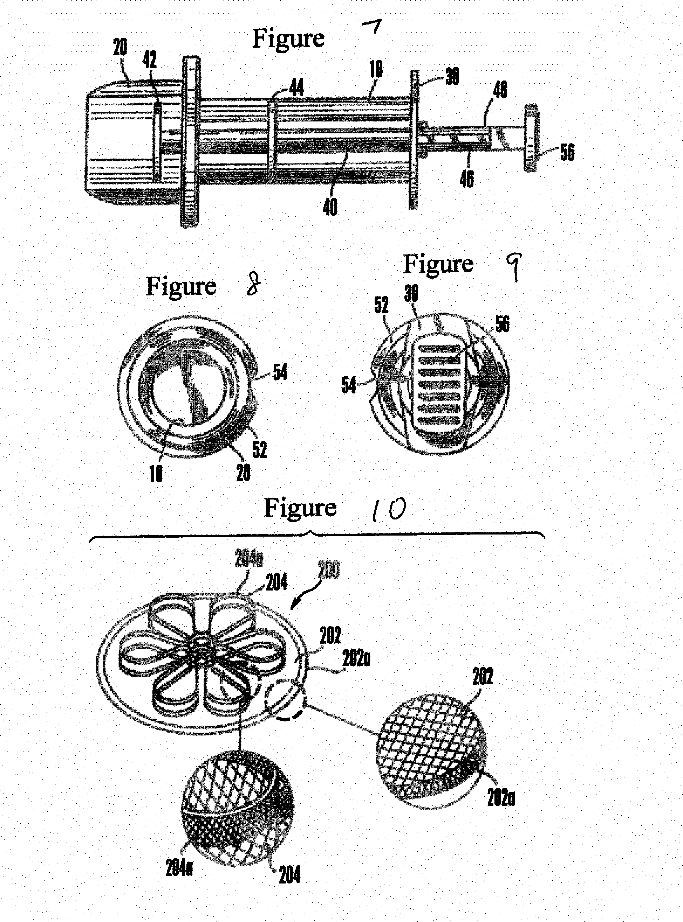

Referring to Figures 3-7, a system 10 includes an implant 12 that may be implemented by any of the implants discussed in the above-incorporated patent applications including the implant 200 shown in Figure 10 and discussed below. In some implementations such as for hernia repair, to hold the implant 12 and position it adjacent tissue to be repaired, at which point the implant is ejected onto the tissue, a fibrotic band interrupter and implant introducing device 14 may be provided with a housing 16 that may be made of transparent or translucent plastic. The housing 16 includes a cylindrical hollow proximal barrel 18 and a hollow distal bulb 20, with the bulb 20 and barrel 18 being coaxial as shown and being engaged with each other to define a continuous channel from a proximal end 22 of the barrel 18 to a distal opening 24 of the bulb 20. The channel can have a first diameter in the barrel and a second diameter in the bulb, and the second diameter can be larger than the first diameter.

As also shown, the bulb 20 has a larger outside diameter than the barrel 18 and has a rounded distal segment 26 tapering down to the distal opening 24. Thus, the distal bulb 20 is curved from the open distal end 24 such that a distal diameter of the bulb is smaller than a proximal diameter of the bulb so that the bulb can be advanced into an opening in tissue distal diameter first and then advanced further until the proximal diameter is in the opening so as to gently stretch the tissue during advancement of the bulb, thereby interrupting fibrotic bands in the tissue. Stated differently, the diameter of the distal opening than the largest diameter of the bulb 20.

As best shown in Figure 6, an interior stop 28 is formed in the barrel 18 and extends into the channel formed by the barrel 18 and bulb 20. In the example shown, the interior stop is an annular ring circumscribing the barrel. Also, as best shown in Figures 4 and 5, a closure element 30 covers the proximal end of the barrel 18 and defines a round opening 32 and an open keyway 34 contiguous to the round opening 32. In the example shown, the keyway includes a first rectilinear opening 34 extending radially from the round opening 32 of the closure element 30 and a second rectilinear opening 36 extending radially from the round opening 32 of the closure element 30 and radially opposed to the first rectilinear opening 34.

A plunger 38 including a stem 40 is slidably disposed in the channel from the proximal end of the barrel 18 into the channel, terminating at a distal disc 42. A proximal disc 44 is formed on the stem 40. Also, a key is formed on the stem 40. As best shown in cross-reference to Figures 4, 5, and 6, the key of the plunger 38 in the example shown includes radially opposed first and second ribs 46, 48 on the stem 40 that are slidably disposed in respective rectilinear openings 34, 36 of the keyway.

With the above description in mind, the plunger 38 is movable relative to the housing between a distal position (Figures 3 and 6), in which the distal disc 42 is closely juxtaposed with the distal opening 24 of the bulb 20, and an unlocked proximal position (Figures 4 and 7), in which the proximal disc 44 rearwardly abuts the interior stop 28 to prevent proximal motion of the plunger 38 relative to the housing and in which the key 46, 48 of the plunger 38 aligns with the keyway 34, 36 of the closure element 30 to permit distal motion of the plunger 38 toward the distal position. Still further, the plunger can be rotated to a locked proximal position substantially in the same axial position as shown in Figures 4 and 7 but in which the plunger 38 is at a different angular location relative to the barrel from an angular position of the plunger 38 in the unlocked proximal position such that the key 46, 48 of the plunger 38 is not aligned with the keyway 34, 36 of the closure element 30. Instead, the key abuts the closure element to prevent distal motion of the plunger 38 relative to the housing.

In non-limiting examples, the closure element 30 may include radially opposed flat wings 50 extending radially away from the barrel. These serve the purpose of finger grips which enable single hand operation. Also, a stop flange 52 can circumscribe a proximal end of the bulb 20 and can extend radially outwardly therefrom. As shown, the stop flange 52 can define a circular periphery except for an alignment notch 54 (Figures 4, 5, and 7) formed in the periphery. As will become clearer in light of description below related to the preferred example implant of Figure 10, when properly installed, the notch 54 is aligned with a loop space that is formed in the implant body when the implant is deformed into the deploy configuration in the bulb 20 to facilitate the visualization of juxtaposing the loop space with a spermatic cord of a patient during deployment of the implant from the bulb 20 by aligning the loop space with the notch 54 during installation. A thumb plate 56 (Figure 9) can be disposed on the proximal end of the plunger 38 as shown to receive a user's finger or thumb.

Figure 10 shows an implant device 200 that includes a round deformable thread mesh 202 designed to lay against a muscle wall and a ribbon or thread implant 204 engaged with the mesh 202 and designed to fill a hole in the muscle wall. The implant 204 is formed of a ribbon of mesh strands in a flower petal configuration as shown. The implant 204 alternatively may be a plug disclosed in the present assignee's U.S. patent application serial no. 11/934,897, incorporated herein by reference.

The implant 204 and/or mesh 202 may be provided with strengthening members in accordance with disclosure below. Briefly, in the non-limiting example shown strengthening members 202a may be provided around the periphery of the mesh 202 while strengthening members 204a are provided around the peripheries of the tops and bottoms of each "petal" of the implant 204.

In the example shown, the strengthening members 202a, 204a are established by thread fibers that are more closely knitted together than the fibers of the mesh 202/implant 204. The fibers of the strengthening members 202a, 204a, which individually may be the same size or smaller (e.g., a mil in diameter smaller) than the fibers of the mesh 202 and implant 204, are woven (including as by knitting or sewing) into the fibers of the mesh 202 and implant 204, respectively. This creates additional stiffness by concentrating more material in one area, resulting in increased fiber density, increased thickness, or both.

In example non-limiting embodiments the mesh 202 can be knitted in an open weave pattern, using polypropylene fibers three to eight mils in diameter. The mesh 202 can have a pore size of between eight-tenths of a square millimeter and sixteen square millimeters (0.8mm² - 16mm²). To establish the strengthening member 202a, polypropylene fibers of, e.g., three to eight mils in diameter are sewn around the edge of the mesh 202 in a close knit sewing pattern and/or multiple passes can be made around the edge to build up additional fiber density. For example, the fiber density of the strengthening member 202a may be ten to one hundred times the fiber density of the remainder of the mesh 202.

As an alternate means of construction stiffer regions with additional fibers, additional fiber material can be welded to the mesh material. Similarly to when the additional fibers are woven into the material, the additional fibers are integrated to the mesh material and cannot be easily removed.

For instance, a polypropylene mesh ring can be constructed of fibers three to eight mils in diameter. One to four additional rings, each one-tenth of an inch to a half an inch in width, of the same material and of the same outer diameter as the mesh 202 can then be welded onto the mesh 202. This creates additional fiber density (as viewed from the top) on the edge of the mesh 202, creating a stiffer material in selected locations, biasing the material in a wrinkle free condition.

Likewise, present principles set forth above contemplate that the implant 204 can have both stiffness and elasticity, so that the combination of structure has a resistance to crush, but can still return to an original configuration if deformed. In some embodiments the overall amount of material may be minimized, and the stiffness can be anisotropic. This may be achieved by increasing the fiber density in specific regions in the same manner as described above.

In greater detail, a knitted mesh material can be knitted into a strip with a more open knit in the middle (pore size of between eight-tenths of a square millimeter and sixteen square millimeters (0.8mm² - 16mm²) and significantly greater fiber density (length of fiber in a given area) at the edges (fiber density ten to one hundred times greater than in the base material) using a polypropylene fiber three to eight mils in thickness. This strip can then be heat set into a final weave configuration and further heat set into a petal configuration. This particular method creates resistance to circumferential crush on the sides of the petals, but minimal resistance to crush from the top.

As the fiber thickness increases, the stiffness increases, but the elasticity (i.e. the ability to return to a given shape after being deformed) decreases. Therefore, the amount of fibers and fiber thickness can be established to obtain the desired combination of stiffness and elasticity. Specifically, in example non-limiting embodiments the mesh 202/implant 204 can be knitted of a polypropylene fiber of between four to eight mils in diameter while the fibers that establish the strengthening members 202a, 204a can be one-half mil to three mils smaller in diameter than those used in the mesh 202/implant 204, and can be knitted to the edges of the mesh 202/implant 204 in a denser configuration to produce specific material properties. To increase the resistance to crush from the top, additional fibers may be knitted in a sinusoidal pattern into the middle of the implant 204.

In use, access to the muscle to be repaired is achieved through open surgery, laparoscopic surgery, endoscopic surgery, or natural orifice surgery, as the situation indicates. For example, when the hole 2 or other defect is in a female's pelvis floor or diaphragm, including the levitor ani and coccygeus, natural orifice surgery through the vagina to the site of the defect may be effected. Or, laparoscopic access may be effected. In either case, the implant is advanced through an endoscope or laparoscope to the site of the defect. Laparoscopy can also be used to repair certain hernias such as inguinal canal hernias. Less desirably, open abdomen surgery may be used to access the defect. When the defect is in the anterior abdominal wall, or the diaphragm, laparoscopic access or open abdomen access may be used to access the defect. When the defect is in the femoral canal, endoscopic or open access may be used to access the defect.

Yet again, when hole or defect 2 to be repaired is in a long extremity muscle such as biceps or a calf muscle, open surgery typically may be used to access the defect, although endoscopy may alternatively be used. Further, when the hole 2 is a myocardial defect, open chest access or endoscopic access through, e.g., the vena cava may be used to access the defect.

The same comments above apply to repairing the non-muscle organs discussed previously.

Once access is achieved, in many cases it is desirable to first dilate the defect to break up damaged fibrotic tissue, although for some defects such dilation may not be indicated. When dilation is indicated, a dilator such as the example device 14 discussed above can be advanced to the defect into the defect to urge the muscle wall surrounding the hole or defect outwardly to dilate it.

In one example, the plunger 38 of the device 14 is retracted relative to the barrel 18 and rotated, e.g., counterclockwise on the body to the locked proximal position. The loops of the implant 200 are then manually folded around the central core of the implant. In the folded position, the user inserts loop structures of the implant 200 into the distal end 24 of the bulb 20, aligning the space that is established between the loops by the folding action with the notch 54. This alignment, in the case of indirect inguinal hernia, helps the loops fold open around the spermatic cord when the implant subsequently is pushed out of the bulb 20.

Typically, the hernia or other tissue defect is prepared according to standard hernia technique. A preperitoneal space large enough to accommodate the implant disk can be created using finger guided blunt dissection of the peritoneal sheath from the abdominal wall. In any case, the bulb 20 with implant 200 located therein is advanced into the inguinal defect. As stated above, in the case of indirect inguinal hernia the notch 54 should be aligned with the spermatic cord to allow the implant 200 to unfold around the cord. If present, the cord should remain stretched laterally throughout the implantation procedure.

Note that the implant disk 202 remains distally outside the open end 24 of the bulb 20 as it is advanced. The bulb 20 is carefully advanced into the defect until the disk 202 of the implant 200 is fully behind the muscle wall in the preperitoneal space and the depth stop flange 52 rests against the external border of the muscular wall.

Once the disk 202 of the implant is in the preperitoneal space, the bulb 20 is gently retracted proximally toward the user until a resistance from the disk is felt. This signifies the implant is ready to be deployed. The plunger 38 is then rotated, e.g., clockwise until it stops, i.e., is in the unlocked proximal position shown in Figures 4 and 7. The barrel 18 with bulb 20 is carefully pulled proximally by the user while the angular position of the barrel relative to the abdominal plane is maintained. As the barrel 18 with bulb 20 is withdrawn, the user pushes the plunger 38 to the distal position shown in Figures 3 and 6 to release the implant 200, allowing the core of the implant to remain inside the defect. Following delivery of the implant, the core of the implant will be in the defect. The loops of the implant should be positioned evenly around the defect to form a complete circle. This can be done using surgical forceps. If a spermatic cord is present, care must be taken to ensure it lies between two of the loops. Using clinical judgment, a securing suture(s) may be placed between the loop(s) of the implant and the muscle wall. Monofilament, non-absorbable sutures may be used. Also, using clinical judgment an anterior mesh (not shown) may be added to help to reinforce the repair. The mesh should be secured to the core of the implant with one single monofilament suture.

As discussed above, in some procedures it may not be indicated to dilate the defect prior to repairing it by means of installing the implant in the defect. In such cases, the implant is guided to the defect or hole site through an endoscope channel, laparoscope channel or trocar, or by hand for open surgery procedures. The implant is then disposed in the defect or hole as described above.

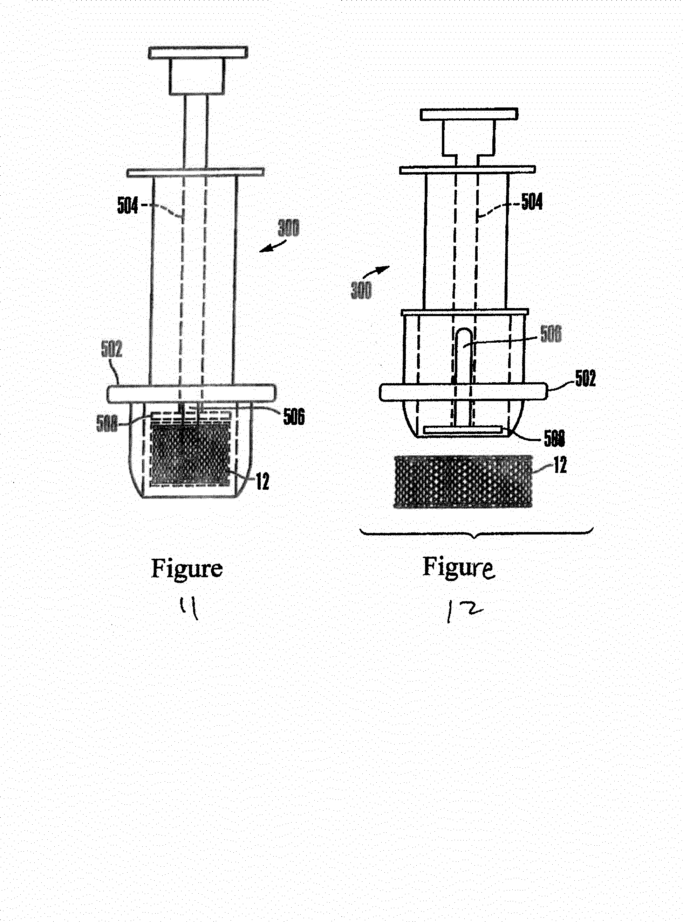

Figures 11 and 12 show an alternate interrupter 300 that is in all essential respects identical in construction and operation to the device 14 described above, except that the stop flange 502 is connected by a connector bar or segment (not shown) to the plunger 504 through a slot 506 such that the stop flange 502 moves with the plunger 504 (and thus with the distal disc 508). This insures that the implant 12 is deployed into the proper plane of the tissue defect and is not pushed distally beyond the proper plane into, e.g., the preperitoneal pocket, since the stop flange 502 will abut intervening tissue before excessive distal pushing occurs.

While the particular SYSTEM AND METHOD FOR REPAIRING MUSCLE DEFECT is herein shown and described in detail, it is to be understood that the subject matter which is encompassed by the present invention is limited only by the claims.

## Claims

1. Method comprising:
providing a mesh body established by plural mesh strands, the mesh body being engaged with at least one strengthening member that is not a mesh strand;
deforming the mesh body to a first configuration in which the mesh body can be advanced into a hole in a body tissue;
advancing the mesh body into the hole; and
allowing the mesh body to assume a second configuration at least partially under influence of the strengthening member in which the mesh body expands to substantially fill the hole, the mesh body creating a scaffold-like structure with a leaf spring-like force to foster tissue ingrowth and
promote tissue regeneration such that new vascular structures can be formed within the mesh while minimizing ingrowth into the mesh body of fibrotic scar tissue, the hole not being an inguinal canal hernia.

2. The method of Claim 1, further comprising dilating the hole prior to advancing the mesh body into the hole.

3. The method of Claim 1, wherein the mesh body is advanced into the hole using open surgery.

4. The method of Claim 1, wherein the mesh body is advanced into the hole using endoscopic surgery.

5. The method of Claim 1, wherein the mesh body is advanced into the hole using laparoscopic surgery.

6. The method of Claim 1, wherein the mesh body is advanced into the hole using natural orifice surgery.

7. The method of Claim 1, wherein the body tissue is defined at least in part by a patient's pelvis floor.

8. The method of Claim 1, wherein the body tissue is defined at least in part by a patient's long extremity muscle.

9. The method of Claim 1, wherein the body tissue is defined at least in part by a patient's heart.

10. The method of Claim 1, wherein the body tissue is defined at least in part by a patient's heart septum.

11. The method of Claim 1, wherein the body tissue is defined at least in part by a patient's abdominal wall.

12. The method of Claim 1, wherein the body tissue is defined at least in part by a patient's diaphragm.

13. The method of Claim 1, wherein the body tissue is defined at least in part by a patient's femoral canal.

14. The method of Claim 1, wherein the body tissue is defined at least in part by a patient's liver.

15. The method of Claim 1, wherein the body tissue is defined at least in part by a patient's kidney.

16. The method of Claim 1, wherein the body tissue is defined at least in part by a patient's lung.

17. The method of Claim 1, wherein the body tissue is defined at least in part by a patient's pancreas.

18. The method of Claim 1, wherein the mesh body is petal-shaped.

19. Method comprising:
providing a mesh body established by plural mesh strands;
deforming the mesh body to a first configuration in which the mesh body can be advanced into a hole in a body tissue;
advancing the mesh body into the hole; and
allowing the mesh body to assume a second configuration in which the mesh body expands to substantially fill the hole, the mesh body creating a scaffold-like structure with a leaf spring-like force to foster tissue ingrowth and promote tissue regeneration such that new vascular structures can be formed within the mesh while minimizing ingrowth into the mesh body of fibrotic scar tissue, wherein the body tissue is defined at least in part by a patient's long extremity muscle, or by a patient's heart, or by a patient's abdominal wall, or by a patient's diaphragm, or by a patient's femoral canal, or by a patient's liver, or by a patient's kidney, or by a patient's lung,
or by a patient's pancreas.

20. The method of Claim 19, wherein the mesh body includes plural petal-shaped lobes.
